# EUROPÄISCHE PATENTSCHRIFT

(11) **EP 1 389 453 B1**
(45) Veröffentlichungstag und Bekanntmachung des Hinweises auf die Patenterteilung: **07.03.2007**
(21) Anmeldenummer: 03000473.3
(22) Anmeldetag: 11.01.2003
(51) Int. Cl.: A61F 5/00

(54) **Band zur Erzeugung einer künstlichen Verengung im Gastro-Intestinal-Trakt**
Band to produce an artificial reduction in the gastrointestinal tract
Anneau pour produire une constriction artificielle du tract gastro-intestinal

(30) Priorität: 16.08.2002 EP 02018449
(43) Veröffentlichungstag der Anmeldung: 18.02.2004
(73) Patentinhaber: AMI Agency for Medical Innovations GmbH, 6840 Götzis (AT)
(72) Erfinder: Höfle, Siegfried, 6800 Feldkirch (AT); Egle, Walter, 6842 Koblach (AT)
(74) Vertreter: Hofmann, Ralf U.

(56) Entgegenhaltungen:
- EP-A- 1 205 148
- FR-A- 2 799 118
- FR-A- 2 802 406
- US-A- 5 152 770

## Beschreibung

Die Erfindung betrifft eine Einrichtung zur Erzeugung einer künstlichen Verengung im Gastro-Intestinal-Trakt mit einem ringförmig um den jeweiligen Teil des Gastro-Intestinal-Trakts legbaren Band, welches einen befüllbaren Hohlraum aufweist, und mit einer Verschlusseinrichtung zum Verbinden der Endbereiche des ringförmig um den Teil des Gastro-Intestinal-Traktes gelegten Bandes.

Eine solche Einrichtung ist beispielsweise in Form eines Magenbandes aus der EP 1 205 148 A1 bekannt. Die Einrichtung weist ein um den Mageneingang legbares Band auf, welches mit einer längsverlaufenden inneren Öffnung ausgebildet ist. Zum irreversiblen Verschließen des um den Mageneingang ringförmig gelegten Bandes weist dieses einen Verschluss mit einem am einen Ende des Bandes angeordneten und eine Einstecköffnung aufweisenden ersten Verschlussteil und einem am anderen Endes des Bandes angeordneten, durch die Einstecköffnung einführbaren und gegenüber dieser verrastbaren zweiten Verschlussteil.

Bei einem weiteren vorbekannten Magenband sind an beiden Enden des Bandes Laschen angeordnet. In der einen Lasche ist eine Durchstecköffnung vorgesehen, durch welche die andere Lasche durchziehbar ist, wobei die freien Enden der beiden Laschen miteinander vernäht werden.

Nach der Anbringung des Bandes um die gewünschte Stelle des Gastro-Intestinal-Traktes, beispielsweise des Mageneingangs, wird zur Ausbildung der gewünschten Verengung Flüssigkeit in den Hohlraum des Bandes eingebracht, wodurch sich die von ihm umgebene Ringöffnung verengt. Neben Magenbändern sind Einrichtungen dieser Art insbesondere auch als Analbänder zum Verschluss eines, gegebenenfalls künstlichen, Anus im Einsatz.

Aufgabe der Erfindung ist es, eine verbesserte Einrichtung der eingangs genannten Art bereitzustellen und erfindungsgemäß gelingt dies durch eine Einrichtung mit den Merkmalen des Patentanspruchs 1.

Die Erfindung basiert auf der Grundidee, durch Verwendung eines Sicherheitsverschlusses, der beim Überschreiten eines vorgegebenen Grenzwertes eines Aufreißzuges öffnet, eine zusätzliche Sicherheit für den Patienten bereitzustellen. Durch die Verwendung eines solchen Sicherheitsverschlusses kann sich das Band im Falle einer zu hohen auf dieses ausgeübten Druckbelastung öffnen, bevor es zu einer Schädigung des der Druckbelastung ausgesetzten Körpergewebes kommt. Hohe Druckbelastungen können beispielsweise im Falle von Magenbändern durch ein Erbrechen des Patienten hervorgerufen werden. Aufgrund der zerstörungsfreien Öffnung des Bandes kann dieses in der Folge durch einen verhältnismäßig einfachen operativen Schritt wieder geschlossen werden, ohne dass ein operativ wesentlich aufwändigerer Austausch der Einrichtung erforderlich wäre.

In einer vorteilhaften Ausführungsform der Erfindung wird die als Sicherheitsverschluss ausgebildete Verschlusseinrichtung von einem eine umfangsgeschlossene Öffnung aufweisenden Steg, der am einen Endbereich des Bandes festgelegt ist, und einem durch die Öffnung im Steg einsteckbaren Fortsatz am anderen Bandende gebildet, welcher mit einem Kragen bzw. Vorsprung zum Hintergreifen des Randes der Öffnung im Steg versehen ist. Durch Verwendung von geeignete Elastizitäten aufweisenden Materialpaarungen für den Steg und den Kragen bzw. Vorsprung sowie eine entsprechende Ausbildung der Geometrie, beispielsweise der Dicke des Steges, kann der gewünschte Grenzwert des Aufreißzuges, oberhalb von welchem die Verschlusseinrichtung öffnet, erreicht werden. Günstigerweise kann hierbei die Dicke des Steges weniger als 5mm, vorzugsweise weniger als 4mm betragen.

In einer vorteilhaften Ausführungsform der Erfindung kann weiters ein vom Fortsatz separates Anschlussröhrchen mit einem inneren Kanal vorgesehen sein, das vom Band in einem Bereich zwischen dessen beiden Enden ausgeht und dessen Kanal mit dem Hohlraum des Bandes verbunden ist. Es wird dadurch die Anbringung des Bandes um das Körperorgan, beispielsweise den Magen, erleichtert und auch eine eventuell erforderliche spätere chirurgische Revision wird erleichtert, wie dies in der Figurenbeschreibung genauer erläutert wird.

Weitere Vorteile und Einzelheiten der Erfindung werden im folgenden anhand der in der beiliegenden Zeichnung dargestellten Ausführungsbeispiele erläutert. In der Zeichnung zeigen:
- Fig. 1: eine Seitenansicht einer erfindungsgemäßen Einrichtung (Blickrichtung B in Fig. 2);
- Fig. 2: eine Ansicht (Blickrichtung A in Fig. 1);
- Fig. 3: eine Stirnansicht des Steges mit der Einstecköffnung, ohne den eingesteckten Fortsatz (Blickrichtung C in Fig. 1);
- Fig. 4: einen Schnitt entlang der Linie AA von Fig. 3;
- die Fig. 5 bis 7: perspektivische Darstellungen der Einrichtung aus verschiedenen Blickwinkeln, wobei das den Steg mit der Einstecköffnung aufweisende Verschlussteil nach Art einer Explosionszeichnung vom Band abgenommen dargestellt ist;
- Fig. 8: eine schematische Darstellung einer Anordnung zur Messung des Aufreißzuges;
- Fig. 9: eine Seitenansicht eines weiteren Ausführungsbeispiels einer erfindungsgemäßen Einrichtung;
- Fig. 10: eine Ansicht;
- Fig. 11: eine Draufsicht;
- Fig. 12: eine perspektivische Darstellung;
- Fig. 13: eine weitere perspektivische Darstellung aus einem anderen Blickwinkel;
- Fig. 14: einen Schnitt entlang der Linie DD von Fig. 10;
- Fig. 15: eine bei der Herstellung der Einrichtung verwendete Verstärkungslage;
- Fig. 16: das Hauptteil der Einrichtung nach dem Spritzgussvorgang und
- Fig. 17 und Fig. 18: das Verschlussteil vor der Anbringung an das Hauptteil in perspektivischen Darstellungen aus verschiedenen Blickwinkeln.

Die Figuren weisen unterschiedlichen Maßstäbe auf. Äquivalente oder zumindest analog wirkende Teile sind in den beiden Ausführungsbeispielen mit den gleichen Bezugszeichen versehen.

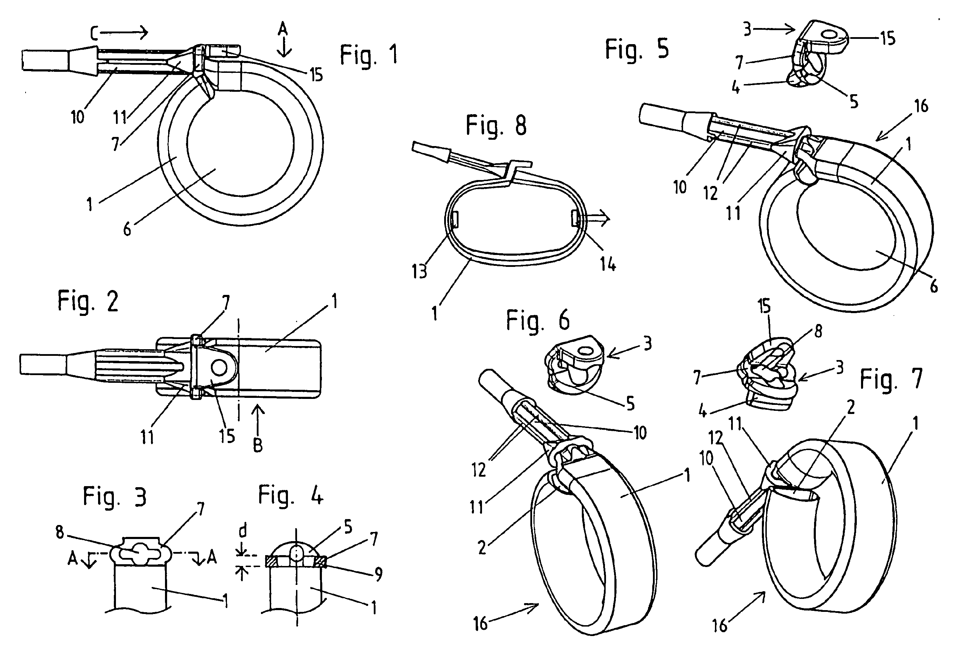

Die beim ersten gezeigten Ausführungsbeispiel (Fig. 1 bis 8) in Form eines Magenbandes ausgebildete Einrichtung weist ein ringförmig um den entsprechenden Teil des Gastro-Intestinal-Traktes, hier den Magen, den Mageneingang oder die Speiseröhre, zu legendes Band 1 beispielsweise aus Silikon auf, das einen in seiner Längsrichtung sich erstreckenden befüllbaren Hohlraum 2 besitzt. Der Hohlraum 2 verläuft über die gesamte Länge dieses schlauchförmigen Bandes 1 durchgehend. Die im ringförmig um das Körperorgan gelegten Zustand des Bandes miteinander zu verbindenden Endbereiche des Bandes sind mit einer Verschlusseinrichtung versehen.

Die erfindungsgemäße Einrichtung ist in diesem Ausführungsbeispiel zweiteilig ausgebildet mit einem Hauptteil 16 und einem mit dem Hauptteil 16 verklebten Verschlussteil 3. Das Hauptteil 16 umfasst das ringförmig um das Körperorgan zu legende schlauchförmige Band 1 und einen am einen Ende diese Bandes 1 angeordneten Fortsatz 10. Der Fortsatz 10 ist röhrchenförmig ausgebildet und weist einen inneren Kanal auf, der mit dem Hohlraum 2 des Bandes 1 verbunden ist.

Wenn die beiden Endbereiches des Bandes 1 über die noch genauer zu erläuternde Verschlusseinrichtung miteinander verbunden sind, umschließt das dann ringförmige Band 1 eine Ringöffnung 6.

Am anderen Ende des Bandes ist das Verschlussteil 3 angeklebt, das auch den Hohlraum 2 im Band 1 endseitig verschließt. Das Verschlussteil 3 weist einen in den Endbereich des Hohlraums 2 des Bandes 1 ragenden Einsteckpfropfen 4 und eine an der endseitigen Stirnfläche des Bandes anliegende Anlageplatte 5 auf, die auch eine endseitige Stirnfläche der Einrichtung bildet, wobei der Einsteckpfropfen 4 und die Anlageplatte 5 mit dem Band 1 verklebt sind.

Von der von der Ringöffnung 6 des Bandes 1 abgewandten Seite der Anlageplatte 5 steht ein Steg 7 nach außen ab, dessen Dicke d günstigerweise kleiner als 5mm, vorzugsweise kleiner als 4mm ist und in welchem eine Einstecköffnung 8 ausgebildet ist. Diese Einstecköffnung 8 wird hier von einer schlitzförmigen Ausnehmung gebildet, in deren mittleren Bereich nach oben und unten ausgehende Verbreiterungen ausgebildet sind. Wie aus Fig. 4 ersichtlich ist, ist der Rand 9 der Einstecköffnung 8 zumindest bereichsweise abgeschrägt, wobei sich die Einstecköffnung zur in Fig. 4 oben liegenden Seite (es ist dies die Einsteckseite für den Fortsatz 10, wie aus dem folgenden noch genauer hervorgehen wird) hin verbreitert. Im gezeigten Ausführungsbeispiel sind diejenigen Abschnitte des Randes 9 mit solchen Abschrägungen versehen, welche in Fig. 3 die linke und die rechte Begrenzung der Einstecköffnung sowie die gesamte untere Begrenzung bilden. Lediglich der Abschnitt, der die obere Begrenzung bildet, weist keine solche Abschrägung auf. Am von der Ringöffnung 6 abgewandten Ende des Steges 7 ist weiters eine Zuglasche 15 angeordnet, die ein Festhalten des Steges beim Verschließen der hier als Magenband ausgebildeten Einrichtung erleichtert.

Auf das freie Ende des Fortsatzes 10 kann ein Ende eines Schlauches aufgesteckt werden, über welchen der Hohlraum 2 des Bandes 1 mit Flüssigkeit befüllbar ist, um den Querschnitt der Ringöffnung 6 entsprechend zu verringern, wobei es sich die der Ringöffnung 6 zugewandte innere Begrenzungswand des schlauchförmigen Bandes nach innen ausdehnt. Die äußere Begrenzungswand ist hierbei vorteilhafterweise mit einer zugfesten Verstärkungslage versehen, um eine Ausdehnung der äußeren Begrenzungswand einzuschränken bzw. zu verhindern. Diese Flüssigkeit kann beispielsweise in herkömmlicher Weise mittels eines Injektionsportes oder mittels einer Pumpeinrichtung eingebracht werden.

Am Fortsatz 10 ist ein Vorsprung 11 angebracht, der im gezeigten Ausführungsbeispiel den Fortsatz 10 allseitig überragt. Die Breite des Vorsprunges 11 ist hierbei auf der der Ringöffnung 6 zugewandten Seite des Fortsatzes am geringsten. Die größte Breite weist der Vorsprung 11 in Teilen seines Abschnitts auf, der sich über die von der Ringöffnung 6 abgewandte Seite des Fortsatzes 10 erstreckt.

Der Vorsprung 11 weist in Richtung zum freien Ende des Fortsatzes 10 eine vom Fortsatz 10 ausgehende und sich konisch verbreiternde Form auf, um ein Durchziehen durch die Einstecköffnung 8 zu ermöglichen. Auf der gegenüberliegenden Seite, welche im eingesteckten Zustand den Rand der Einstecköffnung 8 hintergreift, ist der Vorsprung 11 stufenförmig ausgebildet und steht etwa senkrecht vom Fortsatz 10 ab.

Am schlauchartigen Fortsatz 10 sind weiters vier in Umfangsrichtung jeweils um 90° voneinander beabstandete und in Längsrichtung des Fortsatzes 10 verlaufende, außen abstehende Rippen 12 angeordnet, die einem Abknicken des Fortsatzes 10 und einem damit verbundenen Verschließen seiner Öffnung entgegenwirken.

Zur Ausbildung eines Sicherheitsverschlusses öffnet sich die Verschlusseinrichtung der erfindungsgemäßen Einrichtung bei einem Aufreißzug, der einen Grenzwert überschreitet, wobei dieser Grenzwert im Bereich zwischen 20 und 60 N liegt. Besonders bevorzug ist ein Bereich zwischen 30 und 50 N. Der Aufreißzug wird hierbei in einer Weise gemessen, wie dies schematisch in Fig. 8 dargestellt ist. Die geschlossene Einrichtung wird um Nocken 13, 14 gelegt, von denen mindestens einer verfahrbar ausgebildet ist. Der Abstand der beiden Nocken wird vergrößert, bis sich die Verschlusseinrichtung öffnet. Der halbe Wert der hierfür benötigten Kraft stellt den Aufreißzug der Verschlusseinrichtung dar.

Durch die dargestellte und beschriebene Ausbildung der aus einem elastischen Material, vorzugsweise aus Silikon, gebildeten Verschlusseinrichtung kann sich diese bei einer oberhalb des vorgegebenen Grenzwertes des Aufreißzuges zerstörungsfrei öffnen und in der Folge auch wieder verschlossen werden. Aufgrund der geringsten Breite des Vorsprungs 11 auf der der Ringöffnung 6 zugewandten Seite beginnt das Durchziehen des Vorsprungs 11 durch die Einstecköffnung 8 bei der Einwirkung eines Aufreißzuges in diesem Bereich. Aufgrund der größten Breite des Vorsprunges 11 auf der von der Ringöffnung 6 abgewandten Seite wird der Vorsprung hier zunächst festgehalten. Die seitlichen Bereiche des Vorsprungs, welche die Seiten des Fortsatzes überragen, die in der Richtung der Schmalseiten des Bandes 1 ausgerichtet sind, beginnen sich zunehmend einzurollen und werden mehr und mehr durch die Einstecköffnung durchgezogen, bis auch der von der Ringöffnung abgelegene Abschnitt des Vorsprunges 11 durch die Öffnung durchgezogen wird. Dieser Vorgang wird auch durch die abgeschrägten Abschnitte des Randes 9 der Einstecköffnung unterstützt.

Beispielsweise kann als Material für den Steg 7 und den Vorsprung 11 Silikon mit einer Härte im Bereich von 70 Shore A (+/- 5 Shore A) verwendet werden.

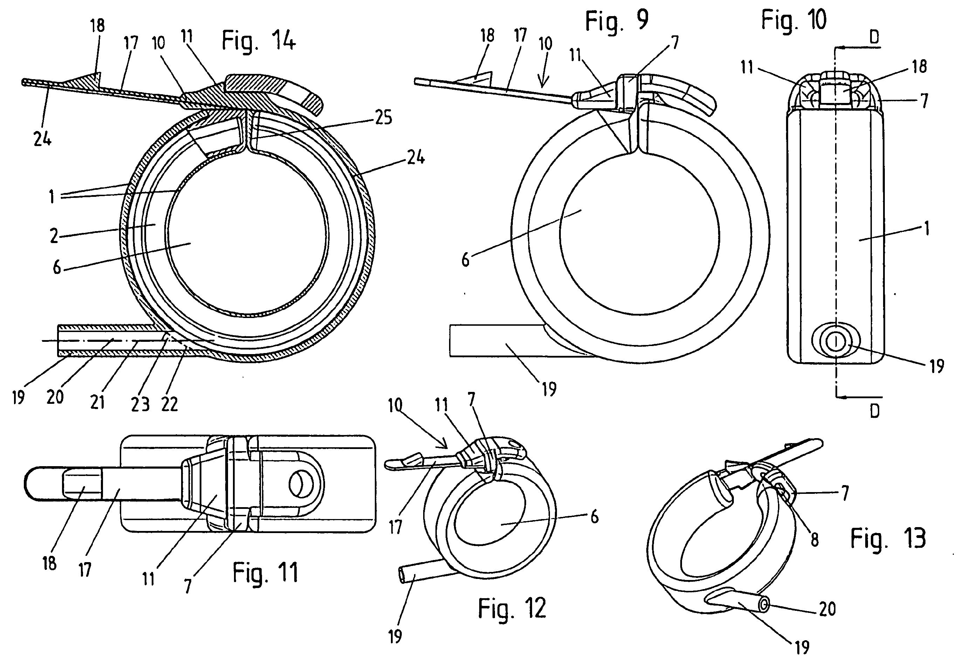

Ein weiteres Ausführungsbeispiel der Erfindung ist in den Figuren 9 bis 18 dargestellt, wobei die Einrichtung wiederum als Magenband ausgebildet ist. Wiederum weist die Einrichtung ein mit einem durchgehenden Hohlraum 2 versehenes Band 1 auf, dessen eines Ende von einem eingeklebten Verschlussteil 3 geschlossen ist und an dessen anderem Ende ein Fortsatz 10 angeordnet ist. Das Verschlussteil 3 und der Fortsatz 10 sind mit zusammenwirkenden Teilen einer Verschlusseinrichtung versehen, in deren geschlossenem Zustand das schlauchförmige Band 1 ringförmig geschlossen ist und eine Ringöffnung 6 umgibt.

Die Verschlusseinrichtung umfasst wiederum einen vom Verschlussteil 3 nach außen abstehenden Steg 7, in dem eine umfangsgeschlossene Einstecköffnung 8 ausgebildet ist, deren Ränder hier aber parallel zueinander verlaufen, sowie einen am Fortsatz 10 angebrachten Vorsprung 11. Zum Schließen der Einrichtung wird der Fortsatz 10 durch die Einstecköffnung 8 gezogen, bis der Vorsprung 11 durch die Einstecköffnung (unter einer elastischen Aufweitung derselben) durchgezogen ist und auf der der Einstiegsseite gegenüberliegenden Seite des Steges 7 die Ränder der Einstecköffnung 8 übergreift. Der Vorsprung 11 überragt hier den Fortsatz 10 nur auf der von der Ringöffnung abgewandten Seite des Fortsatzes 10 und an den beiden in Richtung der Schmalseiten des Bandes ausgerichteten Seiten des Fortsatzes 10. Auf der Innenseite des Fortsatzes 10, also in Richtung zur Ringöffnung 6 ist der Fortsatz in diesem Bereich dagegen glatt ausgebildet.

Zur Erleichterung des Schließens des Bandes ist der Fortsatz 10 mit einer Zuglasche 17 versehen, und zwar in einem vom Vorsprung 11 aus gesehen in einem in Richtung zum freien Ende des Fortsatzes 10 liegenden Abschnitt desselben. Die Zuglasche 17 weist einen Vorrasthaken 18 auf.

Bei diesem Ausführungsbeispiel ist der Fortsatz 10 nicht mit einem den Fortsatz 10 durchsetzenden Kanal versehen, sondern es ist ein vom Fortsatz separates Anschlussröhrchen 19 vorgesehen, das einen inneren Kanal 20 aufweist, der mit dem Hohlraum 2 des Bandes 1 verbunden ist. Das Anschlussröhrchen geht vom Band im Bereich zwischen dessen beiden Enden aus, wobei die Längsachse 21 des Anschlussröhrchens mit der Umfangslinie 22 des Bandes einen Winkel 23 von weniger als 60°, vorzugsweise von weniger als 45° einschließt. Das Anschlussröhrchen 19 steht somit mehr oder weniger tangential vom Band 1 ab.

Weiters weist die Einrichtung einen Verstärkungslage 24 auf, die sich durch die äußere Wand des Bandes 1 über die gesamte Länge des Bandes 1 und durch den Fortsatz 10, vorzugsweise bis zu dessen freien Ende, durchgehend erstreckt.

Diese Verstärkungslage 24 weist in Längsrichtung und vorteilhafterweise auch in Querrichtung über ihre gesamte Ausdehnung durchgehende Fäden auf und kann insbesondere als Rechteckgewebe, beispielsweise aus einem Kunststoffmaterial ausgebildet sein. Diese Verstärkungslage verstärkt einerseits die äußere Wand des Bandes 1, um bei der Befüllung des Bandes eine Ausdehnung der äußeren Wand zu verhindern, und andererseits die Verbindung zwischen dem Band und dem Fortsatz 10 sowie weiters die Zuglaschen 17 gegenüber einer Zugbeanspruchung, wie sie bei der Anbringung der Einrichtung und bei deren Verschluss ausgeübt wird.

Vorteilhafterweise besteht das Band 1 aus einem weicheren Material als das Endstück 3 und der Fortsatz 10 mit den daran angeordneten Komponenten der Verschlusseinrichtung. Beispielsweise kann das Band eine Härte im Bereich von 20 bis 40 Shore A aufweisen und die Teile der Verschlusseinrichtung eine Härte im Bereich von 70 Shore A. Alle Teile der Einrichtung, mit Ausnahme der Verstärkungslage, können aus Silikon bestehen.

Der Fortsatz 10 setzt sich im Bereich der äußeren Wandung des Bandes 1 über das Ende des Bandes hinaus fort. Die auf der Seite des Fortsatzes 10 den Hohlraum 2 des Bandes 1 verschließende endseitige Stirnwandung 25 springt somit gegenüber dem Fortsatz 10 nach innen vor und dieses Stirnwandung besteht ebenfalls aus dem weichen Material des Bandes 1.

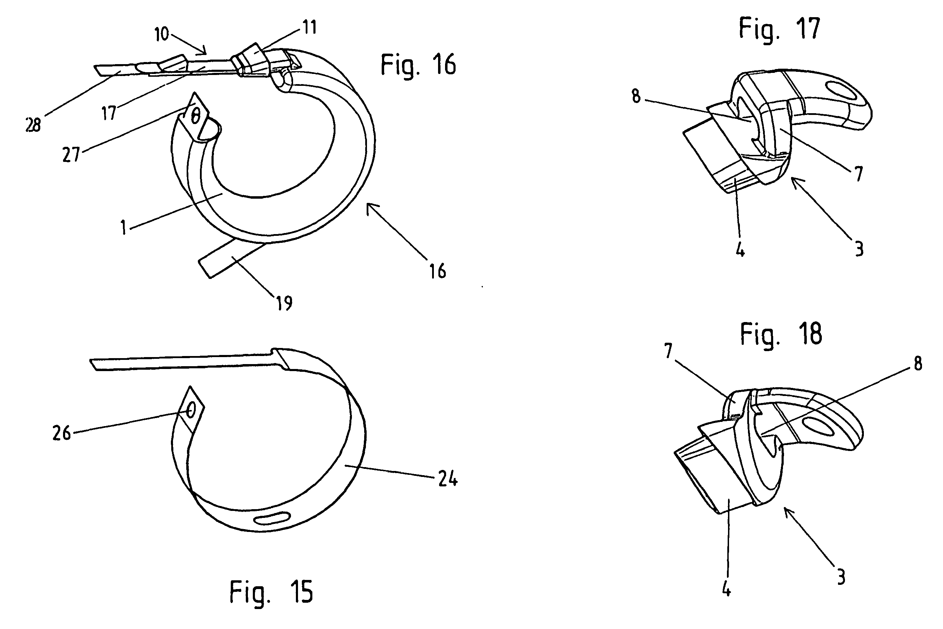

Zur Herstellung des Bandes wird die in Fig. 15 dargestellte Verstärkungslage mit ihrer Öffnung 26 in einen Kern eingehängt, der in einem vom Hohlraum angeordnet ist. Nach dem Einspritzen des Kunststoffes in den Formhohlraum und dem Herausziehen des Kernes aus dem Hohlraum 2 des Bandes erhält man das in Fig. 16 dargestellte Hauptteil, wobei die über die Zuglasche 17 und das Bandende vorstehenden Abschnitte 27, 28 der Verstärkungslage noch abgeschnitten werden. In der Folge wird das in den Fig. 17 und 18 dargestellte Verschlussteil 3 mit seinem Einsteckpfropfen 4 in den Hohlraum 2 an den Fortsatz 10 gegenüberliegenden Ende des Bandes 1 eingeschoben und mit dem Band verklebt.

Zur Anbringung des Magenbandes um den Magen wird nach der entsprechenden Präpäration der Operationsstelle das Band um den Magen herumgezogen, wobei eine Operationszange an der Zuglasche 17 angreift. Das Röhrchen 19 muss dabei nicht um den Magen herumgezogen werden und es erfolgt bei der Operation auch keine Zugbeanspruchung dieses Röhrchens 19, sodass dieses aus einem weichen, körperfreundlichen Material ausgebildet werden kann, beispielsweise mit der gleichen Härte wie das Band 1 und vorzugsweise ebenfalls aus Silikon. Beim Herumziehen des Bands um den Magen wird die Gefahr von Verletzungen von Blutgefäßen stark vermindert, da diesem Herumziehen keine vorspringenden scharfen Kanten entgegenwirken. Der Vorrasthaken 18 und der Vorsprung 11 fallen in Richtung zum freien Ende des Fortsatzes 10 in konisch ab und die Stirnwandung 25 besteht aus einem weichen nachgiebigen Material und kann sich somit leicht in Richtung zur Außenwandung des Bandes 1 hin abbiegen.

Nach dem Herumziehen des Bandes um den Magen wird die Zuglasche 17 in die Einstecköffnung 8 eingeführt bis zunächst der Vorrasthaken 18 am Rand der Einstecköffnung 8 einhängt. In der Folge werden Operationszangen sowohl an der Zuglasche 17 als auch an der Zuglasche 15 angesetzt und der Vorsprung 11 wird durch die Einstecköffnung 8 durchgezogen, sodass die Verschlusseinrichtung verrastet ist. Wie im Zusammenhang mit dem ersten Ausführungsbeispiel beschrieben, ist die Verschlusseinrichtung als oberhalb eines vorgegebenen Grenzwertes eines Aufreißzuges selbstöffnender Sicherheitsverschluss ausgebildet.

Es kann vorkommen, dass eine spätere chirurgische Revision d.h. eine Neuplatzierung des Bandes erforderlich ist, beispielsweise wenn das Band verrutscht ist oder eine Magenerweiterung in einem anderen Bereich sich gebildet hat, zu der das Band versetzt werden soll. Hierbei kann die als Sicherheitsverschluss ausgebildete Verschlusseinrichtung des Magenbandes durch Aufbringung eines entsprechenden Zuges zerstörungsfrei geöffnet werden und das Band vom Magen abgenommen werden und an der neuen Stelle wiederum um den Magen gelegt und geschlossen werden. Da das Röhrchen getrennt von der Verschlusseinrichtung am Band angeordnet ist, kann das Röhrchen 19 kann hierbei mit dem Injektionsport verbunden bleiben, sodass eine zweite Operationsstelle beim Injektionsport vermieden werden kann. Das Röhrchen 19, das in den Figuren nur als kurzes Rohrstück dargestellt ist, kann wesentlich länger ausgebildet sein und durchgehend bis zum Injektionsport reichen, sodass die Anbringung eines Zwischenstückes (mit der Möglichkeit von Undichtheiten oder einem sich Lösen der Verbindung) entfällt.

Unterschiedliche Modifikationen der gezeigten Ausführungsbeispiele der Erfindung sind denkbar und möglich, ohne den Bereich der Erfindung zu verlassen. In Abhängigkeit vom verwendeten Material kann beispielsweise die Dicke d des Steges 7, die im gezeigten Ausführungsbeispielen etwa 3mm beträgt, oder die Breite des Vorsprungs auf den unterschiedlichen Seiten des Fortsatzes verändert werden.

Eine erfindungsgemäß Einrichtung könnte weiters nicht nur als Magenband sondern beispielsweise auch zum Verschluss eines künstlichen oder natürlichen Darmausgangs eingesetzt werden, wobei zum Schließen und Öffnen des Darmausgangs Flüssigkeit mittels eines (herkömmlichen) Reservoirs mit veränderbarem Volumen in das Band eingebracht bzw. aus diesem abgelassen werden kann.

### Legende zu den Hinweisziffern:

- 1: Band
- 2: Hohlraum
- 3: Verschlussteil
- 4: Einsteckpfropfen
- 5: Anlageplatte
- 6: Ringöffnung
- 7: Steg
- 8: Einstecköffnung
- 9: Rand
- 10: Fortsatz
- 11: Vorsprung
- 12: Rippe
- 13: Nocken
- 14: Nocken
- 15: Zuglasche
- 16: Hauptteil
- 17: Zuglasche
- 18: Vorrasthaken
- 19: Anschlussröhrchen
- 20: Kanal
- 21: Längsachse
- 22: Umfangslinie
- 23: Winkel
- 24: Verstärkungslage
- 25: Stirnwandung
- 26: Öffnung
- 27: Abschnitt
- 28: Abschnitt

## Patentansprüche

1. Einrichtung zur Erzeugung einer künstlichen Verengung im Gastro-Intestinal-Trakt mit einem ringförmig um den jeweiligen Teil des Gastro-Intestinal-Trakts legbaren Band (1), welches einen befüllbaren Hohlraum (2) aufweist, und mit einer Verschlusseinrichtung zum Verbinden der Endbereiche des ringförmig um den Teil des Gastro-Intestinal-Traktes gelegten Bandes (1), **dadurch gekennzeichnet, dass** die Verschlusseinrichtung als Sicherheitsverschluss ausgebildet ist, der sich bei einem einen Grenzwert überschreitenden Aufreißzug zerstörungsfrei öffnet und der wiederverschließbar ist, wobei dieser Grenzwert des Aufreißzuges im Bereich zwischen 20 und 60 N, vorzugsweise zwischen 30 und 50 N liegt.

2. Einrichtung nach Anspruch 1, **dadurch gekennzeichnet, dass** die als Sicherheitsverschluss ausgebildete Verschlusseinrichtung einen eine umfangsgeschlossene Einstecköffnung (8) aufweisenden Steg (7), der am einen Endbereich des Bandes (1) festgelegt ist, und einen durch die Einstecköffnung (8) im Steg (7) einsteckbaren Fortsatz (10) aufweist, der vom anderen Endbereich des Bandes ausgeht und mit einem Vorsprung (11) zum Hintergreifen des Randes (9) der Einstecköffnung (8) im Steg (7) versehen ist.

3. Einrichtung nach Anspruch 2, **dadurch gekennzeichnet, dass** der Steg (7) an einem Verschlussteil (3) angeordnet ist, das an einem Endbereich des Bandes (1) mit diesem verklebt ist und auch den Hohlraum (2) des Bandes (1) an diesem Ende verschließt.

4. Einrichtung nach Anspruch 2 oder Anspruch 3, **dadurch gekennzeichnet, dass** der Steg (7) und der Vorsprung (11) aus Silikon bestehen, welches vorzugsweise eine Härte im Bereich von 70 Shore A aufweist.

5. Einrichtung nach einem der Ansprüche 2 bis 4, **dadurch gekennzeichnet, dass** der Vorsprung (11) zumindest über die von der Ringöffnung (6) abgewandte Seite des Fortsatzes (10) und die beiden anschließenden, in Richtung der Schmalseiten des Bandes (1) ausgerichteten Seiten des Fortsatzes (10) vorspringt.

6. Einrichtung nach Anspruch 5, **dadurch gekennzeichnet, dass** die Breite des Vorsprungs im Bereich der der Ringöffnung (6) zugewandten Seite des Fortsatzes (10) schmaler als im Bereich der übrigen Seiten ist oder die der Ringöffnung (6) zugewandte Seite des Fortsatzes (10) im Bereich des Vorsprungs (11) im Wesentlichen glatt ausgebildet ist.

7. Einrichtung nach Anspruch 5 oder Anspruch 6, **dadurch gekennzeichnet, dass** die Breite des Vorsprungs im Bereich der von der Ringöffnung (6) abgewandten Seite des Fortsatzes (10) zumindest teilweise breiter ist als im Bereich der in Richtung der Schmalseiten des Bandes (1) ausgerichteten Seiten des Fortsatzes (10).

8. Einrichtung nach einem der Ansprüche 2 bis 7, **dadurch gekennzeichnet, dass** der Vorsprung auf der dem freien Ende des Fortsatzes (10) zugewandten Seite sich ausgehend vom Fortsatz (10) konisch verbreiternd ausgebildet ist.

9. Einrichtung nach einem der Ansprüche 2 bis 8, **dadurch gekennzeichnet, dass** der Rand (9) der Einstecköffnung (8) zumindest bereichsweise abgeschrägt ist, wobei sich die Einstecköffnung (8) zur Einsteckseite für den Fortsatz (10) hin verbreitert.

10. Einrichtung nach einem der Ansprüche 2 bis 9, **dadurch gekennzeichnet, dass** die Dicke (d) des Steges (7) kleiner als 5mm, vorzugsweise kleiner als 4mm ist.

11. Einrichtung nach einem der Ansprüche 2 bis 10, **dadurch gekennzeichnet, dass** ein vom Fortsatz (10) separates Anschlussröhrchen (19) mit einem inneren Kanal (20) vorgesehen ist, das vom Band (1) im Bereich zwischen dessen beiden Enden ausgeht und dessen Kanal (20) mit dem Hohlraum (2) des Bandes (1) verbunden ist.

12. Einrichtung nach Anspruch 11, **dadurch gekennzeichnet, dass** die Längsachse des Anschlussröhrchens (19) mit der Umfangslinie (22) des Bandes (1) einen Winkel von weniger als 60°, vorzugsweise von weniger als 45° einschließt.

13. Einrichtung nach Anspruch 11 oder Anspruch 12, **dadurch gekennzeichnet, dass** das Anschlussröhrchen (19) näher bei demjenigen Ende des Bandes angeordnet ist, an dem der Steg (7) der Verschlusseinrichtung liegt.

14. Einrichtung nach einem der Ansprüche 11 bis 13, **dadurch gekennzeichnet, dass** der Fortsatz (10) mit einer gegenüber dem Vorsprung weiter vom Band (1) beabstandeten Zuglasche (17) versehen ist, welche vorzugsweise einen Vorrasthaken (18) aufweist.

15. Einrichtung nach einem der Ansprüche 2 bis 14, **dadurch gekennzeichnet, dass** die äußere Wand des Bandes (1) und der Fortsatz (10) mit einer durchgehenden, zugfesten Verstärkungslage (24) versehen sind, die sich vorzugsweise über die gesamte Länge des Bandes (1) und des Fortsatzes (10) erstreckt.

16. Einrichtung nach Anspruch 15, **dadurch gekennzeichnet, dass** die Verstärkungslage (24) in Längsrichtung, vorzugsweise auch in Querrichtung, über die Ausdehnung der Verstärkungslage (24) durchgehende Fäden aufweist.

17. Einrichtung nach einem der Ansprüche 2 bis 16, **dadurch gekennzeichnet, dass** die auf der Seite des Fortsatzes (10) den Hohlraum (2) des Bandes (1) verschließende endseitige Stirnwandung (25) des Bandes (1) gegenüber dem Fortsatz (10) nach innen vorspringt und aus einem weichen Material, vorzugsweise mit einer Härte im Bereich von 20 bis 40 Shore A ausgebildet ist.

## Claims

1. A device to create an artificial constriction in the gastrointestinal tract, comprising a band (1) which may be placed annularly around the particular part of the gastrointestinal tract, which band has a fillable cavity (2), and a closure device for joining the end regions of the band (1) placed annularly around the part of the gastrointestinal tract, **characterised in that** the closure device is a safety closure which opens in a non-destructive manner in the event of a tearing-open tension exceeding a limit value and which is closable again, this limit value of the tearing-open tension lying in the range between 20 and 60 N, preferably between 30 and 50 N.

2. A device according to Claim 1, **characterised in that** the closure device designed as a safety closure comprises a bar (7) having a peripherally closed insertion opening (8), which bar is fixed in place on one end region of the band (1), and an extension (10), insertable through the insertion opening (8) in the bar (7), which extension extends from the other end region of the band and is provided with a projection (11) for engaging behind the edge (9) of the insertion opening (8) in the bar (7).

3. A device according to Claim 2, **characterised in that** the bar (7) is disposed on a closure part (3) which is adhesively bonded at an end region of the band (1) to the said end region and also closes the cavity (2) of the band (1) at this end.

4. A device according to Claim 2 or Claim 3, **characterised in that** the bar (7) and the projection (11) are composed of silicone which preferably has a hardness in the region of 70 Shore A.

5. A device according to one of Claims 2 to 4, **characterised in that** the projection (11) projects at least beyond the side of the extension (10) remote from the annular opening (6) and the two adjoining sides of the extension (10) oriented in the direction of the narrow sides of the band (1).

6. A device according to Claim 5, **characterised in that** the width of the projection is narrower in the region of the side of the extension (10) directed towards the annular opening (6) than in the region of the remaining sides, or the side of the extension (10) directed towards the annular opening (6) is designed so as to be substantially smooth in the region of the projection (11).

7. A device according to Claim 5 or Claim 6, **characterised in that** the width of the projection is at least partially wider in the region of the side of the extension (10) remote from the annular opening (6) than in the region of the sides of the extension (10) oriented in the direction of the narrow sides of the band (1).

8. A device according to one of Claims 2 to 7, **characterised in that** the projection is designed so as to widen conically on the side directed towards the free end of the extension (10), starting from the extension (10).

9. A device according to one of Claims 2 to 8, **characterised in that** the edge (9) of the insertion opening (8) is chamfered at least in regions, the insertion opening (8) widening towards the insertion side for the extension (10).

10. A device according to one of Claims 2 to 9, **characterised in that** the thickness (d) of the bar (7) is less than 5 mm, preferably less than 4 mm.

11. A device according to one of Claims 2 to 10, **characterised in that** a connecting tube (19) is provided, separate from the extension (10), with an internal duct (20), which connecting tube extends from the band (1) in the region between the two ends thereof and the duct (20) of which is connected to the cavity (2) of the band (1).

12. A device according to Claim 11, **characterised in that** the longitudinal axis of the connecting tube (19) forms an angle with the peripheral line (22) of the band (1) of less than 60°, preferably less than 45°.

13. A device according to Claim 11 or Claim 12, **characterised in that** the connecting tube (19) is disposed closer to the end of the band at which the bar (7) of the closure device is located.

14. A device according to one of Claims 11 to 13, **characterised in that** the extension (10) is provided with a pull strap (17) spaced further from the band (1) compared with the projection, which pull strap preferably has a preliminary snap hook (18).

15. A device according to one of Claims 2 to 14, **characterised in that** the outer wall of the band (1) and the extension (10) are provided with a continuous reinforcement layer (24) which has tensile strength and preferably extends over the entire length of the band (1) and of the extension (10).

16. A device according to Claim 15, **characterised in that** the reinforcement layer (24) has filaments extending therethrough in the longitudinal direction, preferably also in the transverse direction, over the extent of the reinforcement layer (24).

17. A device according to one of Claims 2 to 16, **characterised in that** the front wall (25) on the end side of the band (1) closing the cavity (2) of the band (1) on the side of the extension (10) projects inwardly in relation to the extension (10) and is made of a soft material, preferably with a hardness in the region of 20 to 40 Shore A.

## Revendications

1. Dispositif de réalisation d'un rétrécissement artificiel dans le système gastro-intestinal comportant une bague annulaire (1) pouvant être placée autour de la partie concernée du système gastro-intestinal, et présentant un espace creux (2) pouvant être rempli, et un dispositif de fermeture pour relier les zones d'extrémité de la bague annulaire (1) pouvant être placée autour de la partie du système gastro-intestinal,
**caractérisé en ce que**
le dispositif de fermeture est configuré en tant que fermeture de sécurité qui s'ouvre sans destruction en cas de traction d'ouverture supérieure à une valeur limite et qui peut être refermée, cette valeur limite de la traction d'ouverture étant comprise entre 20 et 60 N, de préférence entre 30 et 50 N.

2. Dispositif selon la revendication 1,
**caractérisé en ce que**
le dispositif de fermeture configuré en tant que fermeture de sécurité possède une entretoise (7) présentant une ouverture d'insertion (8) fermée en périphérie et fixée sur l'une des zones d'extrémité de la bague (1), et un prolongement (10) pouvant être inséré à travers l'ouverture d'insertion (8) dans l'entretoise, lequel prolongement part de l'autre zone d'extrémité et est pourvu d'une saillie (11) qui vient saisir le bord (9) de l'ouverture d'insertion (8) dans l'entretoise (7) par l'arrière.

3. Dispositif selon la revendication 2,
**caractérisé en ce que**
l'entretoise (7) est disposée sur une pièce de fermeture (3) qui est collée, sur une zone d'extrémité de la bague (1), à celle-ci et ferme également l'espace creux (2) de la bague (1) sur cette extrémité.

4. Dispositif selon la revendication 2 ou la revendication 3,
**caractérisé en ce que**
l'entretoise (7) et la saillie (11) sont constituées de silicone qui a, de préférence, une dureté de l'ordre de 70 Shore A.

5. Dispositif selon l'une quelconque des revendications 2 à 4,
**caractérisé en ce que**
la saillie (11) dépasse au moins du côté du prolongement (10) opposé à l'ouverture annulaire (6) et des deux côtés adjacents du prolongement (10) adjacents, dirigés dans le sens des petits côtés de la bague (1).

6. Dispositif selon la revendication 5,
**caractérisé en ce que**
la largeur de la saillie est plus petite au niveau du côté du prolongement (10) dirigé vers l'ouverture annulaire (6) qu'au niveau des autres côtés ou bien le côté du prolongement (10) dirigé vers l'ouverture annulaire (6) est essentiellement lisse au niveau de la saillie (11).

7. Dispositif selon la revendication 5 ou la revendication 6,
**caractérisé en ce que**
la largeur de la saillie est au moins en partie plus grande au niveau du côté du prolongement (10) opposé à l'ouverture annulaire (6) qu'au niveau des côtés du prolongement (10) dirigés vers les petits côtés de la bague (1).

8. Dispositif selon l'une quelconque des revendications 2 à 7,
**caractérisé en ce que**
la saillie sur le côté dirigé vers l'extrémité libre du prolongement (10) s'élargit en formant un cône à partir du prolongement (10).

9. Dispositif selon l'une quelconque des revendications 2 à 8,
**caractérisé en ce que**
le bord (9) de l'ouverture d'insertion (8) est biseauté au moins à certains endroits, l'ouverture d'insertion (8) s'élargissant alors en direction du côté d'insertion du prolongement (10).

10. Dispositif selon l'une quelconque des revendications 2 à 9,
**caractérisé en ce que**
l'épaisseur (d) de l'entretoise (7) est inférieure à 5 mm, de préférence inférieure à 4 mm.

11. Dispositif selon l'une quelconque des revendications 2 à 10,
**caractérisé en ce qu'**
un petit tube de raccordement (19) séparé du prolongement (10) comportant un canal intérieur (20) part de la bague (1) dans la zone située entre ses deux extrémités, et le canal (20) est relié à l'espace creux (2) de la bague (1).

12. Dispositif selon la revendication 11,
**caractérisé en ce que**
l'axe longitudinal du petit tube de raccordement (19) forme un angle inférieur à 60°, de préférence inférieur à 45°, avec la ligne périphérique (22) de la bague (1).

13. Dispositif selon la revendication 11 ou la revendication 12,
**caractérisé en ce que**
le petit tube de raccordement (19) est plus près de l'extrémité de la bague sur laquelle se trouve l'entretoise (7) du dispositif de fermeture.

14. Dispositif selon l'une quelconque des revendications 11 à 13,
**caractérisé en ce que**
le prolongement (10) est pourvu d'une éclisse de traction (17), située plus loin de la bague (1) que la saillie, laquelle éclisse présente de préférence un crochet de pré-encliquetage (18).

15. Dispositif selon l'une quelconque des revendications 2 à 14,
**caractérisé en ce que**
la paroi extérieure de la bague (1) et le prolongement (10) sont pourvus d'une couche de renfort (24) continue et résistante à la traction qui s'étend, de préférence, sur toute la longueur de la bague (1) et du prolongement (10).

16. Dispositif selon la revendication 15,
**caractérisé en ce que**
la couche de renfort (24) présente, dans le sens longitudinal, de préférence, aussi dans le sens transversal, des fils continus sur l'étendue de la couche de renfort (24).

17. Dispositif selon l'une quelconque des revendications 2 à 16,
**caractérisé en ce que**
la paroi frontale (25) terminale de la bague (1) et fermant l'espace creux (2) de la bague (1) sur le côté du prolongement (10) est en saillie vers l'intérieur par rapport au prolongement (10) et est constituée d'une matière tendre, ayant de préférence une dureté de l'ordre de 20 à 40 Shore A.
